Europäisches Patentamt

European Patent Office

Office européen des brevets

(11) Publication number: 0 373 107 B1

# EUROPEAN PATENT SPECIFICATION

(45) Date of publication of patent specification: 23.03.94    (51) Int. Cl.⁵: D21C 9/10, C12S 3/08, D21C 5/00

(21) Application number: 89810889.9

(22) Date of filing: 21.11.89

(54) Use of enzymes of Aureobasidium pullulans in pulp bleaching.

(30) Priority: 23.11.88 US 276080

(43) Date of publication of application:
13.06.90 Bulletin 90/24

(45) Publication of the grant of the patent:
23.03.94 Bulletin 94/12

(84) Designated Contracting States:
CH DE FR GB IT LI SE

(56) References cited:
US-A- 4 830 708

BIOTECHNOLOGY AND BIOENGINEERING.
vol. 32, July 1988, NEW YORK, US, pp. 235
-239; Paice M.G. et al.: "Viscosity-enhancing
bleaching of hardwood kraft pulp with
xylanase from acloned gene"

(73) Proprietor: SANDOZ AG
Lichtstrasse 35
CH-4002 Basel(CH)

(84) Designated Contracting States:
CH FR GB IT LI SE

(73) Proprietor: SANDOZ-PATENT-GMBH
Humboldtstrasse 3
D-79539 Lörrach(DE)

(84) Designated Contracting States:
DE

(72) Inventor: Farrell, Roberta Lee
177 Hobart Street
Danvers, MA 01923(US)
Inventor: Skerker, Paul Steven
1079 Commonwealth Avenue
Apartment 348
Boston, MA 02215(US)

**Description**

This invention relates to the use of the enzymes of the yeast-like fungus Aureobasidium pullulans to reduce the lignin content of wood pulps.

Wood is a complex material which is composed of cellulose, hemicellulose and lignin along with other minor components. The lignin is associated with and even covalently bound to a matrix of cellulose and hemicellulose. In paper making processes, lignin should be removed from the wood material since it reduces the strength, confers a brownish colour and imparts other undesirable characteristics to the finished product. Conventionally, wood chips are first treated with sodium sulphide ($Na_2S$) and sodium hydroxide (NaOH) to degrade lignin substantially. This is called the sulphate or Kraft process. Alternatively other treatments may be of use e.g. the sulphite process. The pulps obtained therefrom are called "chemical pulps".

Chemical pulp e.g. Kraft pulp usually contains about 4-12% by weight of residual lignin which gives the pulp a characteristic brown colour. At this stage of delignification, the Kappa number which reflects the lignin content of the pulp is usually from 10 to 45, more frequently from 12 to 40.

To further reduce the lignin content, pulp is subjected to a series of chemical treatments commonly referred to as bleaching. Many industrial bleaching processes already exist but almost all of them include at least some of the following steps, in various orders:

C      Chlorination with chlorine (in an acidic medium)

$C_D$      Reaction with a mixture of chlorine and chlorine dioxide

D      Reaction with chlorine dioxide (in an acidic medium)

E      Alkaline extraction

$E_O$      Alkaline extraction in the presence of oxygen at an increased pressure

H      Reaction with hypochlorite

P      Reaction with a peroxide.

Most commonly, a bleaching process starts with a chlorine treatment followed by extraction of the chlorinated lignin compounds (C-E stage). In addition to this, the process usually comprises 3 or 4 reaction steps which are necessary to obtain a pulp of a desired brightness level. A typical process consists of the CEHDED or CEDED sequence. (For further description of conventional bleaching process, see Smook, G.A. (1982) Handbook for pulp and paper technologists, TAPPI, Atlanta, GA.)

However, the chlorine treatment is highly undesirable because the effluents resulting from the C-E stage (called E-1 effluents) contain a very large number of chlorinated organic compounds e.g. chlorinated phenolics which are hazardous for the environment. Also, due to their highly corrosive nature, it is quite difficult to recycle the effluents. Thus, from the environmental point of view, it is clear that new techniques for bleaching which may reduce pollution, are highly desirable.

In nature, there exist a number of microorganisms which selectively delignify wood, and degrade and modify lignin. The enzymes involved in such a digestion belong to the classes of oxidases, peroxidases and hemicellulases. Attempts have been made to treat pulp with fungi, such as white-rot fungi or the enzymatic systems obtained therefrom, so as to reduce the lignin content. However, such treatments have undesirable side-effects in that they are damaging to the cellulose. Indeed, the microorganisms secrete not only lignin-degrading enzymes but also cellulases which adversely affect the cellulose content of pulp.

The article of M.G. Paice, R. Bernier, Jr. and L. Jurasek titled "Viscosity-Enhancing Bleaching of Hardwood Kraft Pulp with Xylanase from a Cloned Gene" in Biotechnology and Bioengineering, Vol. 32, p. 235-239 (1988), New York discloses a process for bleaching pulp by treating the pulp with Xylanase from a cloned system which is claimed to be free of cellulase. The specific clones of E.coli are obtained from plasmids of bacterial origin and no reliable information is given for a commercially viable process to produce the enzyme preparation.

It has now been found that the hemicellulases of Aureobasidium pullulans are very effective in degrading the hemicellulose content of pulp so that lignin is effectively dislodged from the hemicellulose matrix or rendered releasable. Lignin may then be separated by an appropriate extraction and removed from pulp. Indeed, xylanases are able to catalyse the hydrolysis of both hardwood and softwood hemicellulose which is a polymer of various polysaccharides especially xylans while mannanases catalyze the hydrolysis of mannan-containing carbohydrates, a major component of softwood hemicellulose.

It has further been found that A. pullulans secretes very low levels of cellulases and that a supernatant obtained from a culture of A. pullulans may therefore be used for selectively removing the lignin content of pulp while the cellulose content remains substantially unadversely affected. Therefore, such treatment may be combined with a chemical bleaching process which comprises a reduced number of treatments (less than 5 or 6 as usually incorporated in a conventional process) and in which the amount of chlorine is also

2

reduced since a satisfactory brightness level may still be achieved.

Finally, it has also been found that paper produced from pulp subjected to a biological treatment with enzymes of A. pullulans in combination with chemical bleaching has enhanced qualities over paper produced from conventionally-bleached pulp. This is especially surprising as one would expect xylan removal to result in a decrease in paper properties such as burst and tear indices (see, e.g. Jeffries et al., 1989 Biotechnology in the Pulp and Paper Industry, 4th International Conference, Raleigh, NC p59-60).

Accordingly, the invention provides a process for bleaching pulp which comprises a) treating the pulp with an enzymatic prepartion containing at least one hemicellulase of Aureobasidium pullulans and b) removing the solubilized lignin from the pulp.

Aureobasidium pullulans is a yeast-like fungus which produces hemicellulases including xylanases and mannanases as identified by their activity on xylan and mannan respectively. Strains of A. pullulans which produce relatively high amount of xylanases are e.g. A. pullulans Y-2311 and Y-2311-1, both of which are available from the NRRL Collection, Northern Regional Research Center, U.S. Department of Agriculture, 1815 North University Street, Peoria, Illinois, USA.

By "bleaching process" as used herein is meant a process for improving the whiteness or brightness of wood pulp or both.

The term "solubilizing" as used herein with reference to lignin in pulp is meant to encompass any effect on lignin which results from the degradation or modification of hemicellulose by enzymatic activity. Thus, lignin may be effectively dislodged from the hemicellulose matrix or rendered releasable.

By "lignin" as used herein is meant not only natural, unmodified forms but also the forms as found in chemically treated pulps which are, in whole or in part, chemically modified by various agents such as those used in the Kraft or sulphite pulping process.

By "biological treatment" as used hereinafter is meant a pulp treatment using an enzymatic preparation comprising at least one hemicellulase of A. pullulans.

Any A. pullulans strain may be used in accordance with this invention for providing an enzymatic preparation for treating the pulp. However, preferred strains include those which produce large quantities of hemicellulases, especially those strains which can be characterized as "enzyme overproducers". Over-producer A. pullulans strains are usually white or coloured variants (pink, red, etc.) in comparison with the common blue/black strains. Examples of these strains are described in Leathers, et. al. Biotech. Bioeng. Symp. (1984) 14: 225. Most preferred strains are A. pullalans Y-2311-1 and Y-2311.

For treating hardwood or softwood pulp, preferred strains are those which produce high amounts of xylanases, more preferably endo-xylanases. Alternatively, mannanase-hyperproducing strains may be also advantageously chosen for treating softwood pulp.

According to one aspect of the invention, A. pullalans may be simply cultured within the pulp, preferably after a thorough inoculation of the pulp, for a time sufficient to achieve a desirable effect. The optimal time depends upon a large number of factors but, in general, may vary from 2 to 20 days and the longer treatments may be divided into stages defined by intermediate washing of the pulp followed by a reinoculation with the fungus. Preferably the individual culture periods are from 2 to 12 days, more preferably from 2 to 8 days. Optimum enzyme production by the preferred strains usually occurs at about 2-4 days from the start of the culture.

According to another aspect of the invention, an enzymatic system containing at least one hemicellulase of A. pullalans may be also of use. While, by "enzymatic system" is meant any kind of enzyme preparation free of viable A. pullalans organisms, for example, those in a purified form or provided as a crude extract of a fungus culture, a preferred type is a supernatant obtained from a culture of A. pullalans. While the supernatant may be used as such, it is preferably concentrated before using it. An advantageous method of concentration involves a simple ultrafiltration conveniently designed to achieve a 5-25 fold concentration and, in any case, to recover a concentrate containing essentially all enzymes which affect lignin or hemicellulose. Such concentrates are still substantially free of any cellulase activity and thus will not damage the cellulose content of pulp.

Most preferably the supernatant is obtained from a culture of A. pullulans which is grown in the presence of a carbon source suitable for inducing or stimulating the production of hemicellulases by the fungus. This preferably includes xylan, xylose, mannan (galactomannan, glucomannan, galactoglucoman-nan), mannose, mechanical and chemical pulps, wood chips, sawdust or other substances which contained or derived from hemicellulose. It has also been found that the carbon source on which the fungus is grown may affect the thermal stability of the hemicellulases produced by the fungus. Therefore, particularly preferred carbon sources are hardwood and softwood Kraft pulp, mannan-containing carbohydrates and xylan, this later being the most preferred.

For use in the process of the invention, it is also preferred to recover a supernatant from a 2-12 day culture of A. pullulans, when the optimal enzyme production is achieved. As a general rule, the enzyme concentration may range from 0.1 to 500 Xylanase Unit/g of oven dry pulp, preferably from 1 to 100 XU/g, more preferably from 15 to 30 XU/g. One xylanase unit corresponds to one $\mu$mol/min of reducing sugar produced from xylan per minute at 50°C.

When using an enzymatic system, the process is preferably carried out at a temperature which enhances the enzymatic activity i.e. from about 10 to about 80°C, more preferably from 20 to 60°C, most preferably from 40 to 50°C. The period of time necessary to achieve a desirable effect depends upon a number of factors. However, good results are indicated to occur within a term of enzymatic treatment which ranges from 1 to 8 hours, preferably from 2 to 5 hours. Although the term may be extended to 30 hours, and even more, this will only bring modest additional benefits.

The process of the invention may be applied to a wide variety of wood pulps the residual lignin content of which is to be reduced. Unbleached wood pulps which may be treated with the process of the invention are advantageously thermomechanical pulps (TMP), chemimechanical pulps (CMP), chemithermomechanical pulps (CTMP) and chemical pulps (CP) such as sulphite and Kraft pulps, these latter being preferred.

Following the initial biological treatment using a culture of A. pullulans with an enzymatic system of A. pullulans, removal of the solubilized lignin from pulp may be carried out either by washing, filtration or by extraction, preferably by extraction. Washing with e.g. water is preferably carried out at a temperature from 40°C to 80°C, more preferably at about 60°C. Suitable conventional extractants include, for example, bases such as alkali metal hydroxides alone or with hydrogen peroxide, dimethylformamide, dioxane, acetone and alcohol. A dilute aqueous sodium hydroxide extraction is generally preferred. A typical extraction step may be carried out at a pulp consistency from 3 to 20%, preferably from 5 to 15% at a temperature between 60 and 80°C. The final pH is above 10.8, preferably above 11, otherwise the removal of lignin is incomplete. Retention time may be from 30 minutes to 3 hours, preferably from 45 minutes to 2 hours.

It is particularly preferred to carry out an extraction e.g. an alkaline extraction in the presence of oxygen or peroxide. Advantageously, oxygen is added under pressure. This is not only beneficial to achieve bleaching but also to enhance the brightness of the pulp.

For upgrading pulp, the process of the invention may be used in combination with other conventional chemical treatments as those described in the introduction i.e. C, $C_D$, D, E, $E_O$, H or P. Thus, a process of the invention may also comprise subjecting the pulp to a pre-treatment or after-treatment with chemical bleaching agents such as extractants or chlorine-containing compounds, e.g. chlorine. Alternatively, the chemical treatment may immediately follow the biological treatment, the removal of the solubilized lignin being achieved later. Advantageously, the chemical treatment is carried out after the biological step so that the chemical bleaching agents do not interfere with the enzymatic activity. The process of the invention is preferably accompanied by a series of 2 or more chemical treatments, more preferably not more than 4, most preferably not more than 3 chemical treatments.

It has further been found that treating the pulp with a culture of A. pullulans or with an enzymatic system of A. pullulans, before subjecting the pulp to a chemical bleaching process has surprising beneficial results in term of reduced amount of chemicals necessary for bleaching.

With regard to a conventional bleaching process, the amount of chlorine-containing compounds (Chl), e.g. chlorine, effective for improving the bleaching degree of pulp is usually determined by the following formula I:

$$\text{Chl [expressed in \% (W/W) of oven dried pulp*]} = 0.2 \times K \qquad (I)$$

in which K is the Kappa number of the pulp to be treated.

For example, if the Kappa number is 15, then the amount of chlorine should be 3%. In a $C_D$ treatment part of the chlorine is replaced by chlorine dioxide. The ratio of chlorine to chlorine dioxide may vary depending on the requirements of the mill, but a typical ratio is 9:1.

If the pulp is treated biologically before being subjected to a C or $C_D$ step, then the amount of chlorine necessary to achieve a desired brightness level may be significantly reduced. The "chlorine demand" is at least 20% less than the amount determined by using Formula I, i.e. less than Chl = 0.2K in which K is the Kappa number of the pulp before the biological treatment. Preferably, the amount can be reduced by at least 20%, and even more preferably by at least 30%, most preferably by about 50%. This is of particular interest because less chlorine in a bleaching process results in less harmful effluents.

* drying is carried out for 2 to 10 hours at about 105°C.

The invention is further illustrated as follows:

**GENERAL PROCEDURES**

A. Culture of Aureobasidium pullulans

A. pullulans strain Y-2311-1 is grown and stored on plates with YM medium when the inoculum is an aliquot of a liquid culture.
YM medium contains:
3 g yeast extract
3 g malt extract
5 g peptone
10 g glucose
20 g agar
1 l distilled water.
When the inoculum is taken off from a solid culture, e.g. a single colony grown on an YM plate, the strain is first cultured in the following liquid basal medium:
0.67% yeast nitrogen base
0.2 % asparagine
0.2 % potassium phosphate (monobasic)
1.0 % glucose
Sterilization is made by filtration through 0.22 micrometer filter.
For large scale cultures, the carbon source (glucose) of the YM medium is replaced with 1% xylan, 1% mannan, or 1 % kraft pulp (oven dry weight). The large scale medium is inoculated with an aliquot of a culture grown in basal medium at 1% concentration (vol/vol). The cultures are grown for 2-8 days at 28-30°C. Xylanase activity is typically measured after 2 days. After centrifugation of the culture, the supernatant is recovered. When pulp is used as carbon source, then the pulp consistency of the culture medium is conveniently 1%. [pulp consistency is determined by a standard procedure as the dry weight of the pulp (after drying for 2 to 10 hours at about 105°C) expressed in percentage of the total system measured in volume]

B. Determination of Kappa number

The extent of delignification of the pulp may be indicated by the Kappa number as measured in a standard method described in TAPPI Test Methods (Tappi, Atlanta, Ga.) Vol. 1, 1988 "Kappa number of pulp - T 236 cm 85". The Kappa number is the volume (in millilitres) of 0.1N potassium permanganate solution consumed by one gram of moisture-free pulp (oven dried pulp; drying is carried out for 2 to 10 hours at about 105°C) under the conditions specified in the above method. The results are corrected to 50% consumption of the permanganate added. A lower Kappa number is desirable as it indicates that a smaller amount of lignin is present in pulp.

C. Enzyme preparation

After centrifugation of a culture at 5000 rpm for 20 minutes, the supernatant is collected and optionally concentrated 10-fold using YM-10 Amicon ultrafiltration. The concentrate is then dialyzed against deionized water for 24-48 hours. Enzymes may be stored at 4°C for several weeks or at -70°C for longer periods of time.

D. Assays to determine enzyme activities

1. Endo-xylanase activity:

5 and 10 $\mu$l of an appropriately diluted enzyme concentration is mixed with 50 mM sodium citrate buffer, pH 4.8 (buffer A). This is added to 250 $\mu$l substrate (1% oat spelt xylan in buffer A) preincubated to 50°C. The reaction is allowed to proceed for 30 minutes at 50°C. 0.5 ml of DNSA solution (see below) is then added to the enzyme-substrate solution and heated for 5 minutes at 100°C. 5 ml of deionized water is then added and the absorbance is determined at 540 nm. From a calibration curve using xylose as a standard, the amount of reducing sugars is determined. 1 unit of endo-xylanase activity is defined as 1

5

micromole of reducing sugar produced per min. at 50°C in a 30 minute assay.

DNSA solution:

1 g 3,5-dinitrosalicylic acid
1.6 g sodium hydroxide
30 g sodium potassium tartrate
100 ml deionized water

2. Endo-cellulase activity:

The same procedure is used as in the endo-xylanase assay, except that 0.5% carboxymethyl cellulose (CMC) is used as substrate in place of 1% xylan. When assays are run for 30 minutes no measurable endo-cellulase activity can be associated with the enzymes produced by A. pullulans. Low amounts of activity are detected only after a longer period of time (17 hours).

3. Mannanase activity:

This assay is identical to the assay established for measuring the endo-xylanase activity, except that galactomannan or other mannan-containing carbohydrates is used in place of xylan.

4. Xylosidase, glucosidase and arabinosidase activities:

50 $\mu$l of enzyme are added to 150 $\mu$l of buffer A. This is added to 100 $\mu$l of a 10 mM substrate solution (see below) and incubated at 37°C for 10 minutes. 0.5 ml of sodium carbonate (1 M) is then added to the samples and the absorbance is read at 400 nm. 1 unit of activity is defined as micromoles of p-nitrophenol released per minute at 37°C.
The substate may be:
p-Nitrophenyl-$\beta$-D-xylopyranoside (xylosidase activity)
p-Nitrophenyl-$\beta$-D-glucopyranoside (glucosidase activity)
p-Nitrophenyl-$\beta$-D-arabinopyranoside (arabinosidase activity).

Example 1: **Bleaching of northern softwood kraft pulp using a supernatant obtained from a culture of A. pullulans**

2 gram oven dry weight samples of northern softwood kraft pulp are washed with deionized water, filtered and then suspended in 80 ml of buffer A. The enzymatic preparations used in the study are produced using xylan, northern hardwood kraft pulp, or northern softwood kraft pulp as carbon source for culturing A. pullulans and concentrated as described previously. The samples are treated with an aliquot of the enzymatic solution for 15.5 hours, washed with 1 liter of deionized water, and then treated for a second time with the same amount of enzyme for 5 hours. Following the second enzymatic treatment, the samples are extracted with 80 ml of 2.5% (w/v) sodium hydroxide for 90 minutes at 60°C and then washed with approximately 2.5 liters of deionized water to neutral pH. Kappa numbers of pulps are presented below.

| Sample (Carbon Source) | Enzyme Load* | Kappa |
|---|---|---|
| Control | 0 | 20.2 |
| A. pullulans (Northern Hardwood) | 100 | 18.6 |
| A. pullulans (xylan) | 100 | 18.5 |
| A. pullulans (Northern Softwood) | 10 | 19.3 |

* units of endo-xylanase activity per gram oven dry pulp.

Example 2: **Bleaching of softwood kraft pulp using xylanase and mannanase**

2 gram oven dry weight samples of northern softwood kraft pulp are washed with deionized water, filtered and then suspended in 80 ml buffer A. The enzymatic preparations used in this study are produced as described previously using as carbon source for culturing A. pullulans either xylan, for inducing xylanase production, or galactomannan for inducing mannanase production. The samples are incubated in the presence of xylanase, or xylanase and mannanase for 18 hours at 50°C in water shaking bath and then washed with 1 liter deionized water, followed by extraction with 2.5% (w/v) NaOH at 60°C for 90 minutes, and then washed with 2 liters deionized water. A control sample is also prepared. Kappa numbers are presented below.

| (Units/gram pulp) | | Kappa Number |
|---|---|---|
| Xylanase | Mannanase | |
| 0 | 0 | 19.5 |
| 100 | 0 | 17.9 |
| 100 | 15 | 16.0 |

Example 3: **Bleaching of hardwood kraft pulp using an A. pullulans supernatant**

2 gram oven dry weight samples of northern hardwood kraft pulp are washed with deionized water, filtere and then suspended in 80 ml sodium hydroxide (2.5% w/v) for 60 minutes at 60°C. The samples are then washed with deionized water to neutral pH, and then suspended in 80 ml buffer A. The enzymatic preparation used in this study is produced as described previously using northern hardwood kraft pulp as carbon source. An aliquot of this enzymatic preparation is added to the pulp suspension. After 7 hours at 40°C, the mixture is washed with 1 liter of deionized water, and then treated for a second time with the same amount of enzyme for 15 hours at 40°C. Following the second enzymatic treatment the samples are extracted with 80 ml of 2.5% (w/v) sodium hydroxide for 90 minutes at 60°C. The samples are then washed with 2.5 liters of deionized water to neutral pH. Kappa numbers are presented below.

| Sample | Enzyme load* | Kappa Number |
|---|---|---|
| Control | 0 | 12.3 |
| A. pullulans (Northern Hardwood) | 15 | 8.6 |
| A. pullulans (Northern Hardwood) | 50 | 8.2 |
| A. pullulans (Northern Hardwood) | 100 | 7.8 |

* Units of endo-xylanase activity per gram oven dry pulp.

Example 4: **Growth of A. pullulans on different carbon sources**

500 ml cultures of A. pullulans are grown for 3 days at 28°C in basal medium using as carbon source 1% oat spelt xylan, 1% northern softwood kraft pulp (Kappa number: 22.6) or 1% northern hardwood kraft pulp (Kappa number: 18.6). After three days, the cultures are centrifuged at 5000 rpm for 2 hours and the supernatants are removed, concentrated ten-fold using a YM-10 Amicon ultrafiltration membrane, and then dialyzed for at least 24 hours against deionized water. In parallel, the pulps used as carbon sources are extracted with 80 ml 2.5% sodium hydroxide for 90 minutes at 60°C and then washed with deionized water to neutral pH.

The Kappa number of the hardwood kraft pulp decreases from an initial value of 18.6 to 7.6. If the same type of pulp is not treated by A. pullulans but only extracted, then the Kappa number only decreases from 18.6 to 11.4. After extraction, the Kappa number of the softwood kraft pulp decreases from an initial value of 22.6 to 15.2 when treated first with A. pullulans (rather than from 22.6 to 18 in the absence of initial biological treatment).

2 gram dry weight samples of hardwood kraft pulp are washed with deionized water, filtered, and then suspended in 80 ml buffer A. 2 ml of a 10-fold concentrated enzymatic preparation prepared as above is added to these suspensions. A control sample (without enzyme) is also prepared. All the samples are incubated for 18 hours at 45-50°C, then extracted with 80 ml 2.5% sodium hydroxide for 90 minutes at 60°C and washed with deionized water to neutral pH.

While the control sample has a Kappa number of 10.3, pulp samples enzymatically treated have a Kappa number of 6.7 or of 7.4 depending whether the supernatant is obtained from a culture of A. pullulans grown on hardwood pulp or on xylan, respectively.

Correlations between carbon sources and enzyme activities are summarized below.

| Carbon Source | Protein (mg/ml) | Enzyme Activities (units/ml) | |
| --- | --- | --- | --- |
| | | endo-xylanase | endo-cellulase* |
| xylan | 0.739 | 157 | 0.019 |
| hardwood kraft pulp | 0.719 | 85 | 0.010 |
| softwood kraft pulp | 0.189 | 15 | not determined |

* In a 17 hour assay.

Example 5: **Growth of A. pullulans on kraft pulp.**

500 ml cultures of A. pullulans are grown on either 1% hardwood kraft pulp (Kappa number: 18.6) or 1% softwood kraft pulp (Kappa number: 22.6) in basal medium for 2 to 8 days at 30°C. At a specified day, a culture is filtered to remove the pulp, the supernatant is concentrated ten-fold using a YM-10 Amicon ultrafiltration membrane, then dialyzed against deionized water for at least 24 hours and tested for the protein content and the endo-xylanase and xylosidase activities. The pulp that is filtered out is extracted with 80 ml 2.5% (w/v) sodium hydroxide at 60°C for 90 minutes and then washed with deionized water to neutral pH. The Kappa number of the pulp is determined. The results are given below.

### Northern Softwood Kraft Pulp as Carbon Source

| Day | Protein (mg/ml) | Enzyme Activities (units/ml) | | Kappa number |
| --- | --- | --- | --- | --- |
| | | endo-xylanase | xylosidase | |
| 2 | 0.394 | 3.3 | 0.024 | 16.9 |
| 4 | 0.299 | 6.5 | 0.028 | 16.2 |
| 6 | 0.384 | 3.9 | 0.030 | 18.4 |
| 8 | ND | ND | ND | 15.7 |

Initial Kappa number = 22.6
Kappa number of a pulp sample extracted with sodium hydroxide without

being used as a substrate for A. pullulans = 18.0.

### Northern Hardwood Kraft Pulp as Carbon Source

| Day | Protein (mg/ml) | Enzyme Activities (units/ml) | | Kappa number |
|-----|-----------------|------------------------------|------------|--------------|
| | | endo-xylanase | xylosidase | |
| 2 | 0.678 | 86.7 | 0.051 | 8.6 |
| 4 | 1.613 | 118.2 | 0.118 | 9.6 |
| 6 | 1.200 | 66.0 | 0.120 | 9.6 |
| 8 | ND | ND | ND | 8.6 |

Initial kappa number = 18.6
Kappa number of a pulp sample extracted with sodium hydroxide without being used as a substrate for A. pullulans = 11.4.

ND – not determined

### Example 6: Stability of the enzymes produced by A. pullulans when grown on different substrates

The enzymatic activities of the supernatants obtained from cultures of A. pullulans grown either on xylan or on northern hardwood kraft pulp are tested for their stability at 50°C. Aliquots of supernatants are first incubated at 50°C and then immediately assayed at 50°C for both endo-xylanase activity and xylosidase activity.

The enzymes produced when xylan is the carbon source have a higher thermal stability than the enzymes produced when kraft pulp is the carbon source. When xylan is the carbon source, the endo-xylanase activity remains at 100% of the original activity after exposure to 50°C for 30 hours. The xylosidase activity decreases to 94% of the initial activity. Conversely, the enzyme produced when hardwood kraft pulp is used as substrate has only 17% of its original endo-xylanase activity after exposure to 50°C for 30 hours and 90% of its original xylosidase activity.

### Example 7: Delignification of kraft pulp at high pulp consistency

2 grams (dry weight) of northern hardwood kraft pulp are washed with deionized water and then filtered dried. Buffer A and xylanase (100 units per gram of dry pulp) are added to give a pulp consistency of 10%. Samples are incubated at room temperature for 7 days. The samples are then extracted with 2.5% (w/v) NaOH for 90 minutes at 60°C and washed with deionized water to neutral pH. A control sample (no addition of xylanase) is prepared and run using the same procedure.

Kappa number for the control sample is 14.4. Kappa number for the pulp sample enzymatically treated is 11.4.

### Example 8:

Identical pulp samples (Kappa number = 15) are treated according to various bleaching sequences (see table below). The "chlorine demand" is first determined conventionally according to formula I, based on the Kappa number of the untreated pulp. The amount of chlorine which is actually added to the reaction medium is lower than the amount calculated by formula I, except for test control 1. The enzymatic treatment is carried out at a xylanase dose of 43 XU/g for 23 hours. At the end of the bleaching sequence, the brightness of the pulp is measured according to the TAPPI standard test method T452. A pulp suitable for use in paper industry should have a brightness level of 88 or higher.

Then the bleached pulp samples are used for manufacturing paper, according to the same process for all the samples. Tear factor, burst breaking strength and breaking length of paper are measured by TAPPI test method T414, T403 and T404 respectively. The higher they are, the better.

The results are to be seen in the Table below.

|  | Control 1 | Control 2 | Exp.1 | Exp. 2 | Exp. 3 |
|---|---|---|---|---|---|
| Bleaching sequence | $C_DE_ODED$ | $BE_OC_DE_OD$ | $XEC_DE_OD$ | $XE_OC_DE_OD$ | $XE_OC_DE_OD$ |
| % of chlorine added in $C_D$ step | 3.0 | 2.10 | 2.25 | 1.65 | 1.50 |
| Final Brightness | 88.9 | 86.2 | 89.4 | 89.1 | 87.8 |
| Burst Factor | 7.1 | 8.9 | 10.8 | 10.8 | 10.7 |
| Tear Factor | 62.8 | 71.0 | 70.7 | 75.0 | 78.4 |
| Breaking Length | 958 | 1039 | 1146 | 1154 | 1195 |
| B: treatment with Buffer only | | | | | |
| X: enzymatic treatment using xylanase of A. pullulans. | | | | | |

Example 9

Experiment 2 of Example 8 is repeated twice using 1.8% of chlorine, the term of the enzymatic treatment being reduced to 6 and 3 hours. Brightness of the pulp is 88.1 and 89.3, respectively.

## Claims

1. A process for bleaching pulp which comprises
   a) treating the pulp with an enzymatic preparation containing at least one hemicellulase of Aureobasidium pullulans and
   b) removing the solubilized lignin from the pulp.

2. A process according to claim 1 in which the enzymatic preparation is obtained from a culture of A. pullulans grown in the presence of a carbon source able to induce or to stimulate the production of at least one hemicellulase by the fungus.

3. A process according to claim 1 or 2 in which the enzymatic preparation is a supernatant obtained from a culture of A. pullulans.

4. A process according to any one of claims 1 to 3 in which the enzymatic preparation contains at least one endo-xylanase of A. pullulans.

5. A process according to any one of claims 1 to 4 in which A. pullulans is the strain Y-2311 or Y-2311-1.

6. A process according to any one of claims 1 to 5 which comprises removing the solubilized lignin by alkaline extraction.

7. A process according to claim 6 which comprises removing the solubilized lignin by alkaline extraction in the presence of oxygen.

8. A process according to any one of claims 1 to 7 which comprises subjecting the pulp to at least one and not more than 3 chemical treatments using chemical bleaching compounds.

9. A process according to claim 8 which comprises after-treating the pulp with an amount of a chlorine-containing compound at least about 20 % lower than the amount calculated by formula (I)

Chl [expressed in % (weight/weight) of oven dried pulp] = 0.2K     (I)

in which K is the Kappa number of the pulp before the biological treatment.

**10.** A process according to any one of claims 1 to 9 for treating chemical pulp to reduce its lignin component content comprising contacting the pulp with an enzyme preparation produced and isolated from a culture of Aureobasidium pullulans grown in the presence of a carbon source to produce endoxylanase, for a time sufficient to releasably degrade the lignin component of the pulp, and extracting the treated pulp to remove the releasably degraded lignin component, said enzyme preparation being substantially free of cellulases which damage the cellulose content of the pulp.

**11.** A process according to claim 10 in which the carbon source used for the production of endoxylanase is xylan or xylose.

**Patentansprüche**

**1.** Verfahren zum Bleichen von Papierbrei mit den Verfahrensschritten, dass
a) der Papierbrei mit einer enzymatischen Zubereitung, die mindestens eine Hemicellulase von Aureobasidium pullulans enthält, behandelt und
b) das löslich gemachte Lignin vom Papierbrei entfernt wird.

**2.** Verfahren nach Anspruch 1, in weichem die enzymatische Zubereitung aus einer Kultur von A. pullulans erhalten wird, die in Gegenwart einer Kohlenstoffquelle wachsen gelassen wird, welche die Herstellung von mindestens einer Hemicellulase durch den Pilz induziert oder stimuliert.

**3.** Verfahren nach Anspruch 1 oder Anspruch 2, in welchem die enzymatische Zubereitung ein Überstand von einer Kultur von A. pullulans ist.

**4.** Verfahren nach einem der Ansprüche 1 bis 3, in welchem die enzymatische Zubereitung mindestens eine Endoxylanase von A. pullulans enthält.

**5.** Verfahren nach einem der Ansprüche 1 bis 4, in welchem A. pullulans der Stamm Y-2311 oder Y-2311-1 ist.

**6.** Verfahren nach einem der Ansprüche 1 bis 5, welches die Entfernung des löslich gemachten Lignins durch alkalische Extraktion umfasst.

**7.** Verfahren nach Anspruch 6, welches die Entfernung von löslich gemachtem Lignin durch alkalische Extraktion in der Gegenwart von Sauerstoff umfasst.

**8.** Verfahren nach einem der Ansprüche 1 bis 7, dadurch gekennzeichnet, dass der Papierbrei mindestens einer und nicht mehr als drei chemischen Behandlungen unter Verwendung von chemischen Bleichverbindungen ausgesetzt wird.

**9.** Verfahren nach Anspruch 8, welches die Nachbehandlung des Papierbreis mit einer Menge einer chlorhaltigen Verbindung umfasst, die mindestens ungefähr 20% niedriger ist als die Menge, die durch die Formel (I) berechnet wird

Chl[ausgedrückt in %(Gewicht/Gewicht) an ofengetrocknetem Papierbrei] = 0,2K     (I)

in welcher K die Kappa-Zahl des Papierbreis vor der biologischen Behandlung bedeutet.

**10.** Verfahren nach einem der Ansprüche 1 bis 9 zur Behandlung von chemischem Papierbrei, um dessen Ligninkomponentengehalt zu reduzieren, welches umfasst, dass der Papierbrei mit einer Enzymzubereitung, die von einer Kultur von Aureobasidium pullulans produziert und isoliert wurde, welche in Gegenwart einer Kohlenstoffquelle wachsen gelassen wurde, um Endoxylanase herzustellen, genügend lange in Berührung gebracht wird, um die Ligninkomponente des Papierbreis unter Freigabe abzubauen, und dass der behandelte Papierbrei extrahiert wird, um die unter Freigabe abgebaute Ligninkomponente zu entfernen, wobei besagte Enzymzubereitung im wesentlichen frei von Cellulasen ist, welche den Cellulosegehalt des Papierbreis angreifen.

**11.** Verfahren nach Anspruch 10, worin die Kohlenstoffquelle für die Herstellung von Endoxylanase Xylan oder Xylose ist.

**Revendications**

**1.** Un procédé de blanchiment de la pâte à papier, qui comprend
a) le traitement de la pâte à papier par une préparation enzymatique contenant au moins une hémicellulase de Aureobasidium pullulans et
b) l'élimination de la lignine solubilisée, de la pâte à papier.

**2.** Un procédé selon la revendication 1, dans lequel la préparation enzymatique est obtenue à partir d'une culture de A. pullulans cultivée en présence d'une source de carbone capable de provoquer ou de stimuler la production d'au moins une hémicellulase par le champignon.

**3.** Un procédé selon la revendication 1 ou 2, dans lequel la préparation enzymatique est un surnageant obtenu à partir d'une culture de A. pullulans.

**4.** Un procédé selon l'une quelconque des revendications 1 à 3, dans lequel la préparation enzymatique contient au moins une endo-xylanase de A. pullulans.

**5.** Un procédé selon l'une quelconque des revendications 1 à 4, dans lequel A. pullulans est la souche Y-2311 ou Y-2311-1.

**6.** Un procédé selon l'une quelconque des revendications 1 à 5, qui comprend l'élimination de la lignine solubilisée par extraction alcaline.

**7.** Un procédé selon la revendication 6, qui comprend l'élimination de la lignine solubilisée par extraction alcaline en présence d'oxygène.

**8.** Un procédé selon l'une quelconque des revendications 1 à 7, qui comprend le fait de soumettre la pâte à papier à au moins un et pas plus de 3 traitements chimiques utilisant des composés pour le blanchiment chimique.

**9.** Un procédé selon la revendication 8, qui comprend le post-traitement de la pâte à papier par une quantité d'un composé contenant du chlore au moins environ 20% inférieure à la quantité calculée par la formule (I)

Chl [exprimé en % (poids/poids) d'une pâte à papier séchée au four] = 0,2K     (I)

où K est le nombre kappa de la pâte à papier avant le traitement biologique.

**10.** Un procédé selon l'une quelconque des revendications 1 à 9 pour le traitement de la pâte chimique afin de réduire sa teneur en composant ligneux, comprenant la mise en contact de la pâte avec une préparation enzymatique produite et isolée à partir d'une culture de Aureobasidium pullulans cultivée en présence d'une source de carbone pour produire une endoxylanase, pendant un temps suffisant pour assurer la dégradation et la libération du composant ligneux de la pâte, et extraction de la pâte traitée pour éliminer le composant ligneux dégradé et libéré, ladite préparation enzymatique étant essentiellement exempte de cellulases qui détériorent la teneur en cellulose de la pâte.

**11.** Un procédé selon la revendication 10, dans lequel la source de carbone utilisée pour la production de l'endoxylanase est le xylane ou le xylose.